# EUROPEAN PATENT SPECIFICATION

(11) **EP 2 089 121 B1**
(45) Date of publication and mention of the grant of the patent: **08.01.2014**
(21) Application number: 07822340.1
(22) Date of filing: 08.11.2007
(51) Int. Cl.: A63B 24/00, A63B 71/06

(54) **Apparatus for motor training and exercise of the human body**
Vorrichtung zum Motorik-training und zum Training des menschlichen Körpers
Appareil pour l'entraînement moteur et l'exercice du corps humain

(30) Priority: 10.11.2006 IT PD20060413
(43) Date of publication of application: 19.08.2009
(73) Proprietor: Khymeia S.R.L., 35027 Noventa Padovana (IT)
(72) Inventor: FURLAN, Roberto, 35138 Padova (IT)
(74) Representative: Modiano, Micaela Nadia
(86) International application number: PCT/EP2007/062036
(87) International publication number: WO 2008/055949

(56) References cited:
- EP-A- 0 993 845
- WO-A-97/04840
- WO-A-98/42413
- US-A- 5 690 582
- US-B1- 6 244 987

## Description

### Technical Field

The present invention relates to an apparatus for motor training and exercise of the human body, particularly for treating disorders of the musculoskeletal system, of the neuromotor system, and disorders linked to cognitive functions (psychological/psychiatric disorders).

### Background Art

The fundamental principles of motor sciences seek to recover corporeity and consider movement as a language of body expression which is destined to affect the psychological and physical growth of a person.

The concept of "psychophysical unity" of the individual is indispensable for the educational process, and reeducational process in the case of the path for recovery from trauma, of a person, especially if one considers the importance of the use and control of one's body in establishing social behaviors and intellectual structures.

To achieve these goals, it is necessary to seek specific learning targets as regards:
- physiological enhancement
- restoration, consolidation and coordination of basic motor patterns
- preparation for sports activity.

Physiological enhancement is to be understood as an improvement of the great vital functions (circulatory and respiratory), of muscle tone and of its subsequent strengthening, and of articular mobility to be maintained and developed over time.

Restoration, consolidation and coordination of basic motor patterns are to be found in the improvement of general postural patterns and of motor patterns, of space-time assessment patterns referred both to oneself and to objects (tools), and of patterns for mental prefiguration of rhythms.

The "basic motor patterns" constitute a pivotal element, which references all the simplest motor skills learned during life: walking, running, balancing, et cetera; the person reaches new, increasingly complex abilities and develops increasingly advanced skills starting from these experiences.

Regular and methodical practice of motor activity, dosed and purposed correctly, is known as exercise when it is intended to increase performance capacity and is known as training when it is intended to learn or relearn, on the part of the individual, postural patterns and motor patterns which are new or not new but at least partly forgotten due to traumas or other disorders.

Exercise and training, in order to be effective, must comply with certain principles:
- continuity: constant exercise, avoiding prolonged interruptions,
- progressiveness of the load: the quantity and intensity of the exercise cannot remain constant over time, on penalty of risking a loss of effectiveness due to the body that adapts to the load of the exercise,
- alternation of work steps and recovery steps: it is important to alternate moments of intense work with others which require more bland commitment,
- multilaterality: exercises must be chosen so as to develop harmonically all the factors of performance capacity,
- individualization: as a differentiation of the times and methods related to practice, which cannot be equal for all trainees but need to be customized.

Learning correct practice and acquiring specific capabilities is the main goal which one must seek through exercise and training.

There are various forms of learning:
- by imitation: when the person, by seeing the exercises performed by others, makes a series of attempts to imitate them,
- by intuition: when faced with the need to overcome an obstacle, the person, while not having a model, considers the possible solutions,
- by comprehension: it is generally more convenient to learn under the guidance of a good teacher in an intelligent manner.

To allow true learning, the following are important:
- motivations: being truly interested in learning is the first condition for success,
- understanding the task: to know what one wishes to learn; this is why it is important to observe carefully the demonstration and learn the exact succession of the actions,
- repeated executions of the exercise: repetition of the exercise or of a movement is the best way to automate it and therefore acquire skill,
- returned information and corrections, i.e., the importance of having feedback to understand how movements just performed were made,
- the psychomotor level, i.e., the degree of dexterity possessed by the person who has to learn, a condition which determines the difference between those who learn sooner and those who learn more slowly,
- gratification: to be understood as the pleasure derived from the activity and the satisfaction for the results achieved.

As for all other types of teaching, in motor sciences, too, one speaks of customized planning, working in terms of individualization, respecting the different times and different skills that characterize each person.

Individualizing teaching means customizing it according to the problems to be solved, to the skill, capacity, interests and styles of learning of a person, placing the educational purposes in the contest of planned paths.

For correct planning of training and exercise, it is necessary:
- to detect the initial situation,
- to formulate the specific learning goals,
- to choose content in relation to the program,
- to choose teaching and methodological strategies,
- to select the means and tools to be used,
- to identify the methods for verification and assessment of learning.

Devices for motor exercise and training of the human body are currently known.

These devices generally comprise:
- a device for acquiring biophysiological data,
- means for processing, storing and summarizing the data,
- and a device for returning information related to the acquired or acquired and processed data to an expert operator, to whom the training/exercise of the user is entrusted.

Known devices are generally structured to acquire, analyze and return a single feedback or no feedback to the user, whilst data and signals are processed to be subjected to the expert operator, for example a physician or other specialist, who follows the user/patient in the use of the device.

In this way, the user has little or no return information available to understand how the movements that have just been performed were made and to apply the appropriate corrections thereto.

Further, known types of apparatus allow user learning either by intuition, i.e., by possessing the luck/skill to determine autonomously the best solution among the ones that are possible, despite not having a model of the correct postural or motor pattern to be achieved, or by comprehension, i.e., on the basis of the teachings of an operator/teacher; but it is not at all granted that all users have the necessary intuition or that there is an operator/teacher to guide and correct the user constantly, where comprehension of the task is a fundamental step in understanding what has to be learnt and how this learning must occur.

Generally, a user can access one of these known apparatuses only by going to a hospital or hospital-like facility, and if the user is a temporarily or permanently disabled individual, a senior citizen or any other person who has difficulties in moving from home to the place where the apparatus is located, continuing a therapy or a training or exercise process can be very inconvenient if not impossible to do.

US 6244987 discloses a wellness system comprising an exercise load providing unit for providing a load of a physical exercise for a user, and for measuring a movement of the user who is doing the physical exercise, a virtual reality environment providing unit for providing the user with a virtual reality environment that can cause user's plural senses such as the sense of sight, hearing, touch, and smell to work, according to the contents of the virtual reality environment, and a control unit for controlling the virtual reality environment provided by the virtual reality environment providing unit according to the user's movement measured by the exercise load providing unit.

### Disclosure of the Invention

The aim of the present invention is to provide an apparatus for motor exercise and training of the human body, particularly for treating disorders of the musculoskeletal system, of the neuromotor system, and of disorders which can be linked to the cognitive functions (psychological/psychiatric disorders), which is capable of obviating the limitations of known apparatuses.

Within this aim, an object of the present invention is to provide an apparatus which facilitates and improves learning on the part of the user of how the movement/exercise is to be performed and of how the movement/exercise has actually been performed.

Another object of the present invention is to provide an apparatus which is capable of providing the user, or an expert operator, with the data and parameters that have been collected and processed by means of a plurality of feedbacks, enhancing the sensory channels for perceiving both the proposed movements and the movements actually performed, thus improving learning.

Another object of the present invention is to provide an apparatus which can be managed by an expert operator even remotely.

Another object of the present invention is to provide an apparatus which can be used autonomously by a user even in the absence of an expert operator.

Another object of the present invention is to provide an apparatus which can be manufactured with known systems and technologies.

This aim and these and other objects, which will become better apparent hereinafter, are achieved by an apparatus for motor training and exercise of the human body, with the features of claim 1.

### Brief Description of the Drawings

Further characteristics and advantages of the invention will become better apparent from the following detailed description of a preferred but not exclusive embodiment thereof, illustrated by way of non-limiting example in the accompanying drawings, wherein:

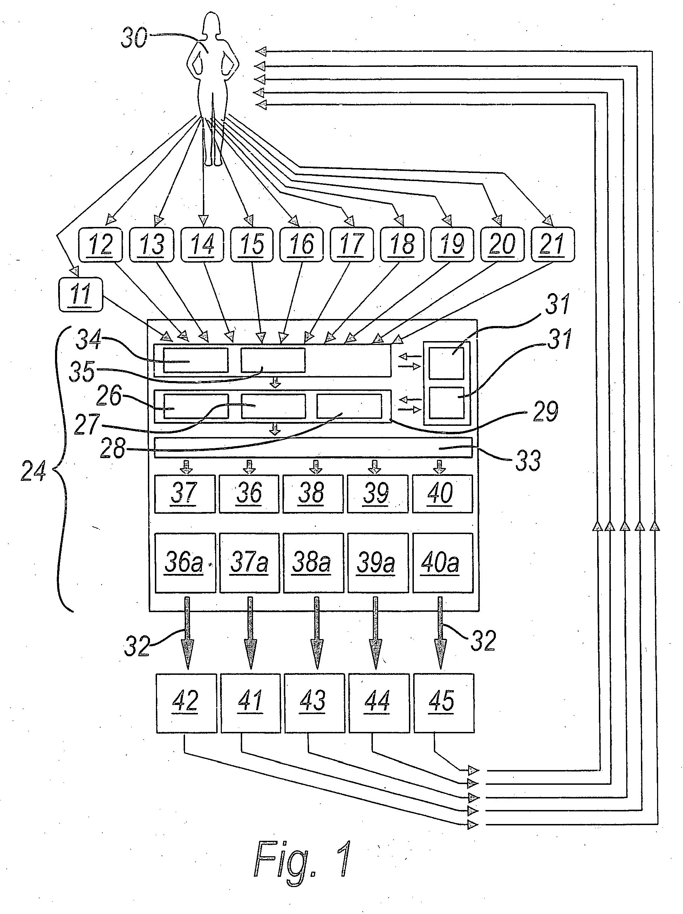

Figure 1 is a schematic view of an apparatus according to the invention,
Figure 2 is a view of an exemplary embodiment of an apparatus according to the invention.

### Ways of carrying out the Invention

With reference to the figures, an apparatus for motor training and exercise of the human body according to the invention is generally designated by the reference numeral 10.

The apparatus 10 comprises
- means for acquiring biophysiological data and motion parameters related to a human action of the type which can be controlled voluntarily by the individual who performs said action,
- means for processing, storing and summarizing the data and parameters that have been acquired,
- and means for returning at least two feedbacks of information related to the acquired, or acquired and processed, data and parameters, suitable to facilitate correction of the voluntary human action being performed, said information return means being designed to utilize at least one sensory channel.

The acquisition means can be constituted by at least one selectively among:
- a device 11 for acquiring parameters related to the position and movement of the user or of a part of the body of the user,
- a device 12 for acquiring electromyographic data,
- a device 13 for acquiring data related to the blood gas saturation level,
- a device 14 for acquiring electroencephalographic data,
- a device 15 for acquiring data related to respiratory activity and to the exchange of gases in the lungs,
- a device 16 for acquiring images,
- a device 17 for acquiring data related to cardiac activity and to blood vessel pressure,
- a device 18 for acquiring data related to electrodermal activity,
- a device 19 for acquiring data related to the noise generated by motion,
- a device 20 for acquiring data related to the consumption of glucose,
- a device 21 for acquiring temperature data.

The device 11 cited above generically for acquiring parameters related to the position and movement of at least one part of the body of the user can comprise one or more sensors of the electromagnetic and/or ultrasound and/or piezoelectric or variable-resistance type and/or based on systems of the accelerometric type.

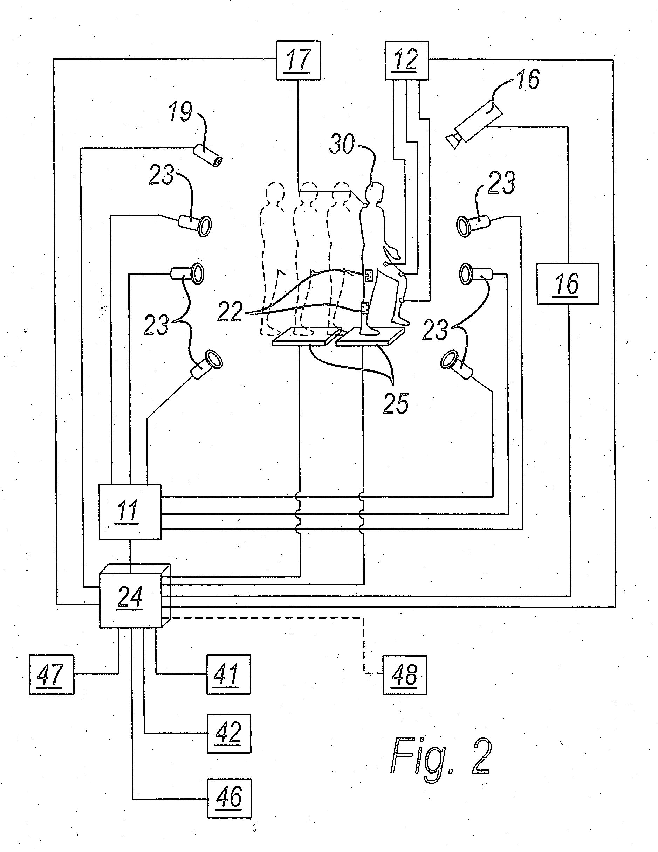

In the exemplary embodiment of Figure 2, the device 11 for acquiring parameters related to the position and motion of at least one part of the body of the user is of the stereophotogrammetric type with optical markers 22 and image analysis; the position and motion of the markers are detected by a series of infrared video cameras 23, which are connected to the central processing unit 24, described in greater detail hereinafter in its components.

The device 11 for acquiring parameters related to the position and motion of at least one part of the body of the user can also be constituted by a GPS (Global Position System) system over radio frequencies if the user, in order to perform his exercise or training, must travel over relatively large distances or in any case over such distances that other types of sensor would be unable to transmit the detected signals to the receiving base.

In a constructive variation of the invention, the device 11 for acquiring parameters related to the position and motion of at least one part of the body of the user comprises photocells and/or contact switches and/or proximity switches, which may also be managed by electromagnetic emission.

The device 11 for acquiring parameters related to the position and movement of at least one part of the body of the user can also be associated with means for studying body balance, as exemplified in Figure 2, which illustrates a dynamometric platform 25 on which a user 30 is required to move (walk, run, jump or other similar actions).

The means for studying body balance can be constituted, alternately or additionally, also by one or more stabilometric platforms, force platforms and the like.

The device 11 for acquiring parameters related to the position and motion of at least one part of the body of the user can also be associated with one or more instruments for analyzing the pressure of the sole of the foot or of parts of the body or of accessories connected to parts of the body.

The means for acquiring motion and position parameters can be associated with gymnasium machines, such as for example a stationary bike provided with control over the number of turns of the pedals, or another generic gymnasium machine of the multifunction or single-function type, which is provided with instruments for measuring motion data related to its parts actuated by a user during exercise.

These means for acquiring motion and position parameters can be constituted, as an alternative, by numeric control machines for motor assistance and rehabilitation.

The temperature data acquisition device comprises contact and/or remote probes or, as an alternative, an instrument for reading the invisible spectrum.

The means for processing, storing and summarizing the acquired data and parameters are constituted by the central processing unit 24 and can comprise, associated with a unit 29 for processing the input data and for modulating the outputs,
- one or more systems or algorithms 26 for correcting the data with respect to environmental and/or circumstantial interference,
- one or more systems 27 for processing signals, data and parameters,
- one or more systems 28 for preparing algorithms for analyzing the acquired data.

Such means for processing, storing and summarizing the acquired data and parameters also comprise:
- one or more systems 31 for storing signals, acquired data, processed data and parameters, such as a hard disk, a memory chip and the like,
- one or more systems (designated schematically by the arrows 32) for data transmission, for example a wired or wireless network,
- one or more systems for managing the output of data, which in turn comprise: the algorithms 33 dedicated to the handling of the signals to be sent to appropriately provided hardware cards, said hardware cards, i.e., a video card 36, an audio card 37, and optional cards for tactile feedback 38, for olfactory feedback 39 and for taste feedback 40, and the corresponding software 36a, 37a, 38a, 39a, 40a for managing said cards,

- one or more systems 34 for analyzing the signals,
- one or more systems 35 for analyzing the data,
- one or more user interface systems, of the type for example based on the Windows® system.

Further, the means for processing, storing and summarizing the acquired data and parameters can also comprise at least one selectively among:
- one or more systems for providing virtual environments,
- one or more systems for virtualizing objects and in general elements which do not exist,
- one or more systems for virtual interaction with objects or real shapes or virtual shapes which are stationary or moving, within virtual environments.

The means for returning at least two feedbacks comprise at least two selectively among
- means 41 for generating visual feedbacks,
- means 42 for generating acoustic feedbacks,
- means 43 for generating tactile feedback,
- means 44 for generating olfactory feedbacks,
- means 45 for generating taste feedback.

Preferably, the delay between the voluntary action and the feedback is such as to allow the user to provide a logical correlation which induces the user to perform a spontaneous correction of the subsequent action or actions.

The faster the feedback, the more the user is facilitated in performing spontaneous correction.

It is also preferable if the indication of the error allows to perceive the correct model or if the correct model is indicated explicitly.

The means 41 for generating visual feedbacks are constituted by at least one among:
- a two-dimensional or three-dimensional display screen,
- stereoscopic goggles,
- a two-dimensional or three-dimensional image projection system,
- other similar systems.

The means for generating visual feedbacks can also comprise
- a system for displaying multidimensional shapes, including anthropomorphic ones, which are adapted to represent the user, and/or any other interacting virtual or automated individual, and/or any other real interacting individual who is the user of an equivalent system by means of another apparatus,
- a system for real-time three-dimensional rendering of objects, optionally also provided with kinematic properties.

The means 42 for generating acoustic feedbacks are constituted by at least one among:
- at least one loudspeaker,
- at least one monaural system,
- mono or stereo headphones,
- a positional audio system.

The means 43 for generating tactile feedback are constituted by at least one among:
- means of the thermal type,
- means of the vibratory type,
- means for force or pressure feedback,
- a series, a network or an array of electrical stimulators applied to particularly sensitive parts of the body,
- air-based or humidified air-based means.

The means of the thermal type are of the type with resistors or Peltier cells or with streams of cold or hot air or the like, while means of the vibratory type can be constituted by sources of vibrations produced by pneumatic or hydraulic pressure waves or eccentric elements actuated by electric motors.

The information return means are adapted to emit feedback to the user 30 of the apparatus and/or to an expert operator to whom the training/exercise of the user is entrusted.

The data processing means are constituted, as mentioned, by a central processing unit 24, which can be controlled directly with an on-site computer 47 or by means of a remote link to a computer or other remote device 46, which is monitored by an expert operator, or for recording the activity of the apparatus 10, a recording which remains available for time-delayed, i.e. not real-time, analysis.

The remote link allows to set up the apparatus 10, for example, even at the user's home, while the expert operator can follow the activities of the user with the apparatus 10 while remaining for example at the clinic or at his/her workplace without having to go to the user to monitor his/her activities.

Remote control can be provided conveniently so that it is performed by means of a data communications network, which is already present in all homes and is available to anyone without excessive additional expenses, or even wirelessly.

The apparatus 10 can also be provided with means for interfacing with one or more portable memories 48, which are available to the user for personalized use of the apparatus 10: the portable memory 48 can contain the training/exercise paths that have been predefined by the expert operator for the user, and the user can perform these activities full autonomously, i.e., without the aid of the presence of such expert operator.

The execution and outcome of the training/exercise session can then be stored in the portable memory 48, available for any use which the user or operator may make of them.

The apparatus 10, in certain configurations, can be enclosed within one or more enclosures which make it ergonomic and convenient to use and carry.

The apparatus 10 according to the invention is well-suited for motor training and exercise of the human body, particularly for treating disorders of the musculoskeletal system, neuromotor system, and disorders which can be linked to the cognitive functions (psychological/psychiatric diseases).

In particular, by way of the possible setup of the apparatus 10 with systems for providing virtual environments or for virtualizing objects and in general elements which do not exist, it becomes possible to approach users affected by disorders of the cognitive functions which can be sensitive to interaction with such virtual environments.

By means of the apparatus 10 it is possible to store the movement sequences to be performed by the user and to propose such recorded movements to the user again so as to induce the user to perform the same movements.

The memories 31 of the processing unit 24 allow, by way of associated systems 28 of analytical algorithms, to select one movement among many, which is assessed by the expert operator or automatically by an appropriately provided algorithm as optimum in order to propose it to the user again.

The apparatus 10 also allows to store, for example by means of a stereophotogrammetric device 11 with optical markers 22, the exact movement of a healthy limb, to be used by reversing it in a mirror-symmetrical manner to propose to the user the optimum movement for the limb with dysfunctions or whose performance is to be improved.

The apparatus 10 also has the advantage of being able to resume and store even very small movements made in the correct direction or in the correct manner and to amplify them with feedback, so that the user has a better perception of the correct direction or of the correct manner in which his/her movement is to proceed.

The apparatus 10 can be programmed and the exercises to be performed can be performed sequentially by the user even in the absence of an expert operator.

The logic of these exercises can be set by the expert operator, while the sequence of exercises is established by one or more of the data analysis systems 28.

As mentioned, to allow true learning, gratification is important, understood as a pleasure deriving from the activity and satisfaction for the results achieved; from this point of view, the apparatus 10 can be provided with a score system, which can be automatically adapted by means of parameters to the performance of the user and is designed to reward the user in proportion to the quality of the exercise performed.

In practice it has been found that the invention thus described solves the problems noted in known types of apparatus for motor training and exercise of the human body, particularly for the treatment of disorders of the musculoskeletal system, neuromotor system, and of disorders which can be linked to the cognitive functions (psychological/psychiatric disorders).

In particular, the present invention provides an apparatus which facilitates and improves learning, on the part of the user, of how the movement/exercise is to be performed and of how the movement/exercise has actually been performed.

Moreover, the present invention provides an apparatus which can provide the user and/or an expert operator with the data and parameters collected and processed by means of a plurality of feedbacks, enhancing the sensory channels for perceiving both the proposed movements and the movements actually performed, thus improving learning.

Further, the present invention provides an apparatus which can be managed by an expert operator even remotely.

Further, the present invention provides an apparatus which can be used autonomously by a user even in the absence of an expert operator.

Moreover, the present invention provides an apparatus which can be manufactured with known systems and technologies.

The invention thus conceived is susceptible of numerous modifications and variations, all of which are within the scope of the appended claims; all the details may further be replaced with other technically equivalent elements.

In practice, the materials employed, so long as they are compatible with the specific use, as well as the dimensions, may be any according to requirements and to the state of the art.

## Claims

1. An apparatus for motor training and exercise of the human body, comprising:
- means (11, 12, 14, 16, 17) for acquiring biophysiological data and motion parameters related to a human action of the type which can be controlled voluntarily by the individual who performs it,
- means (24) for processing, storing and summarizing the acquired data and parameters,
- and means (41, 42, 43) for returning at least two feedbacks of information related to the acquired, or acquired and processed, data and parameters, suitable to facilitate correction of the voluntary human action being performed, said information return means (41, 42, 43) being designed to utilize at least one sensory channel,
said acquisition means (11, 12, 14, 16, 17) comprise at least a device (11) for acquiring parameters related to the position and movement of the user or of a part of the body of the user;
said means (24) for processing, storing and summarizing the acquired data and parameters comprise at least:
- one or more systems for providing virtual environments,
- one or more systems for virtual interaction with real shapes or objects or virtual shapes which are stationary or moving within virtual environments;
said means for returning at least two feedbacks comprise at least
- means (41) for generating visual feedbacks,
- means (42) for generating acoustic feedbacks,
said means (41) for generating visual feedbacks comprise
- a system for displaying multidimensional shapes, including anthropomorphic ones, which are adapted to represent said user, and/or any other interacting virtual or automated individual, and/or any other real interacting individual who is the user of an equivalent system by means of another apparatus,
- a system for real-time three-dimensional rendering of objects, optionally also provided with kinematic properties.

2. The apparatus according to claim 1, **characterized in that** said acquisition means are constituted by at least one selectively among:
- a device (12) for acquiring electromyographic data,
- a device (14) for acquiring electroencephalographic data,
- a device (16) for acquiring images,
- a device (17) for acquiring data related to cardiac activity and to blood vessel pressure,

3. The apparatus according to one or more of the preceding claims, **characterized in that** said device (11) for acquiring parameters related to the position and motion of at least one part of the body of the user selectively comprises: one or more electromagnetic position and motion sensors; or one or more ultrasound position and motion sensors; or a stereophotogrammetric device with optical markers (22) and image analysis; or one or more systems of the accelerometric type; or one or more piezoelectric or variable-resistance sensors.

4. The apparatus according to one of the preceding claims, **characterized in that** said device (11) for acquiring parameters related to the position and motion of at least one part of the body of the user is associated with means for studying body balance.

5. The apparatus according to claim 4, **characterized in that** said means for studying body balance are constituted by a dynamometric platform (25), by stabilometric platforms, force platforms and the like.

6. The apparatus according to one or more of the preceding claims, **characterized in that** said means (11) for acquiring motion and position parameters are associated with gymnasium machines.

7. The apparatus according to one or more of the preceding claims, **characterized in that** said means (11) for acquiring motion and position parameters are associated with numeric-control machines for motion assistance and rehabilitation.

8. The apparatus according to one or more of the preceding claims, **characterized in that** said means (24) for processing, storing and summarizing the acquired data and parameters comprise at least one selectively among:
- one or more systems (27) for processing the signals, the data and the parameters,
- one or more systems (28) for preparing analytical algorithms for the acquired data,
- one or more systems (31) for storing signals, acquired data, processed data and parameters,
- one or more data transmission systems (32),
- one or more systems (33, 36, 36a, 37, 37a, 38, 38a, 39, 39a, 40, 40a) for managing the data output,
- one or more signal analysis systems (34),
- one or more data analysis systems (35),
- one or more user interface systems,
- one or more systems for virtualizing objects and in general elements which do not exist.

9. The apparatus according to one or more of the preceding claims, **characterized in that** said means (41, 42, 43) for returning at least two feedbacks comprise
- means (43) for generating tactile feedback,

10. The apparatus according to one or more of the preceding claims, **characterized in that** said means (41) for generating visual feedbacks are constituted by at least one among:
- a two-dimensional or three-dimensional display screen,
- stereoscopic goggles,
- a two-dimensional or three-dimensional image projection system,
- other similar systems.

11. The apparatus according to one or more of the preceding claims, **characterized in that** said means (42) for generating acoustic feedbacks are constituted by at least one among:
- at least one loudspeaker,
- at least one monaural system,
- mono or stereo headphones,
- a positional audio system.

12. The apparatus according to one or more of the preceding claims, **characterized in that** said means (43) for generating tactile feedback are constituted by at least one among:
- means of the vibratory type,
- means for force or pressure feedback, .
- a series, a network or an array of electrical stimulators applied to particularly sensitive parts of the body.

13. The apparatus according to claim 12, **characterized in that** said means of the vibratory type are sources of vibrations due to pneumatic or hydraulic pressure waves or eccentric elements actuated by electric motors.

14. The apparatus according to one or more of the preceding claims, **characterized in that** said means (24) for processing, storing and summarizing comprise a central processing unit (24) which can be controlled directly or by remote link.

15. The apparatus according to the preceding claim, **characterized in that** said remote control occurs by means of a data communications network which can also be wireless.

16. The apparatus according to one or more of the preceding claims, **characterized in that** it is provided with means for interfacing with one or more portable memories (48) which are available to the user for personalized use of the apparatus.

## Patentansprüche

1. Ein Gerät für Bewegungstraining und -übungen für den menschlichen Körpers Folgendes umfassend:
- Mittel (11, 12, 14, 16, 17) zum Erfassen von biophysiologischen Daten und Bewegungsparametern eine menschliche Handlung der Art betreffend, die von der ausführenden Person willkürlich kontrolliert werden kann,
- Mittel (24) zum Verarbeiten, Speichern und Zusammenfassen der erfassten Daten und Parameter;
- und Mittel (41, 42, 43) zum Liefern von mindestens zwei Informationsrückmeldungen die erfassten bzw. erfassten und verarbeiteten Daten und Parameter betreffend, die dazu geeignet sind, die Verbesserung der willkürlichen menschlichen Handlung, die ausgeführt wird, zu erleichtern, wobei die Informationslieferungsmittel (41, 42, 43) dazu ausgebildet sind, zumindest einen Sinneskanal einzusetzen,
wobei die Erfassungsmittel (11, 12, 14, 16, 17) mindestens eine Vorrichtung (11) zum Erfassen der Parameter die Position und Bewegung des Benutzers oder eines Teils des Körpers des Benutzers betreffend umfassen;
wobei die Mittel (24) zum Verarbeiten, Speichern und Zusammenfassen der erfassten Daten und Parameter mindestens Folgendes umfassen:
- ein oder mehrere Systeme zum Bereitstellen von virtuellen Umgebungen,
- ein oder mehrere Systeme zur virtuellen Interaktion mit realen Figuren oder Objekten oder virtuellen Figuren, die in den virtuellen Umgebungen in Bewegung oder nicht in Bewegung sind;
wobei die Mittel zum Liefern von mindestens zwei Rückmeldungen mindestens Folgendes umfassen:
- Mittel (41) zum Erzeugen visueller Rückmeldungen,
- Mittel (42) zum Erzeugen akustischer Rückmeldungen,
wobei die Mittel (41) zum Erzeugen visueller Rückmeldungen Folgendes umfassen:
- ein System zum Anzeigen multidimensionaler Figuren, einschließlich anthropomorpher Figuren, die dazu geeignet sind, den Benutzer und/oder jede andere interagierende virtuelle oder automatisierte Person und/oder jede andere reale interagierende Person zu repräsentieren, die der Benutzer eines äquivalenten Systems mittels eines anderen Geräts ist,
- ein System zur dreidimensionalen Darstellung von Objekten in Echtzeit, das wahlweise auch über kinematische Eigenschaften verfügt.

2. Das Gerät gemäß Anspruch 1, **dadurch gekennzeichnet, dass** die Erfassungsmittel wahlweise mindestens aus einem der folgenden Elemente bestehen:
- einer Vorrichtung (12) zum Erfassen elektromyographischer Daten,
- einer Vorrichtung (14) zum Erfassen elektroenzephalograpischer Daten,
- einer Vorrichtung (16) zum Erfassen von Bildern,
- einer Vorrichtung (17) zum Erfassen von Daten über die Herzaktivität und den Blutgefäßdruck.

3. Das Gerät gemäß einem oder mehreren der vorangehenden Ansprüche, **dadurch gekennzeichnet, dass** die Vorrichtung (11) zum Erfassen der Parameter die Position und die Bewegung von mindestens einem Teil des Körpers des Benutzers betreffend wahlweise Folgendes umfasst: einen oder mehrere elektromagnetische Positions- und Bewegungssensoren; oder einen oder mehrere Ultraschall-Bewegungs- und Positionssensoren; oder eine stereophotogrammetrische Vorrichtung mit optischen Markierungen (22) und Bildanalyse; oder ein oder mehrere Systeme beschleunigender Art; oder einen oder mehrere piezoelektrische Sensoren oder Sensoren mit variablem Widerstand.

4. Das Gerät gemäß einem der vorangehenden Ansprüche, **dadurch gekennzeichnet, dass** die Vorrichtung (11) zum Erfassen der Parameter die Position und die Bewegung von mindestens einem Teil des Körpers des Benutzers betreffend mit Mitteln zum Ermitteln des Körpergleichgewichts verbunden ist.

5. Das Gerät gemäß Anspruch 4, **dadurch gekennzeichnet, dass** die Mittel zum Ermitteln des Körpergleichgewichts aus einer dynamometrischen Plattform (25), aus stabilometrischen Plattformen, Kraftmessplatten und dergleichen bestehen.

6. Das Gerät gemäß einem oder mehreren der vorangehenden Ansprüche, **dadurch gekennzeichnet, dass** die Mittel (11) zum Erfassen der Bewegungs- und Positionsparameter mit Trainingsgeräten verbunden sind.

7. Das Gerät gemäß einem oder mehreren der vorangehenden Ansprüche, **dadurch gekennzeichnet, dass** die Mittel (11) zum Erfassen der Bewegungs- und Positionsparameter mit NC-Maschinen für die Bewegungsunterstützung und Rehabilitation verbunden sind.

8. Das Gerät gemäß einem oder mehreren der vorangehenden Ansprüche, **dadurch gekennzeichnet, dass** die Mittel (24) zum Verarbeiten, Speichern und Zusammenfassen der erfassten Daten und Parameter wahlweise mindestens eines der folgenden Elemente umfassen:
- ein oder mehrere Systeme (27) zum Verarbeiten der Signale, der Daten und der Parameter,
- ein oder mehrere Systeme (28) zum Vorbereiten analytischer Algorithmen für die erfassten Daten,
- ein oder mehrere Systeme (31) zum Speichern von Signalen, der erfassten Daten, der verarbeiteten Daten und Parameter,
- ein oder mehrere Datenübertragungssysteme (32),
- ein oder mehrere Systeme (33, 36, 36a, 37, 37a, 38, 38a, 39, 39a, 40, 40a) zum Verwalten der Datenausgabe,
- ein oder mehrere Signalanalysesysteme (34),
- ein oder mehrere Datenanalysesysteme (35),
- ein oder mehrere Benutzerschnittstellensysteme,
- ein oder mehrere Systeme zum Virtualisieren der Objekte und im Allgemeinen der Elemente, die nicht existieren.

9. Das Gerät gemäß einem oder mehreren der vorangehenden Ansprüche, **dadurch gekennzeichnet, dass** die Mittel (41, 42, 43) zum Liefern von mindestens zwei Rückmeldungen
- Mittel (43) zum Erzeugen der taktilen Rückmeldung umfassen.

10. Das Gerät gemäß einem oder mehreren der vorangehenden Ansprüche, **dadurch gekennzeichnet, dass** die Mittel (41) zum Erzeugen visueller Rückmeldungen mindestens aus einem der folgenden Elemente bestehen:
- einem 2D- oder 3D-Bildschirm,
- 3D-Brillen,
- einem zweidimensionalen oder dreidimensionalen Bildprojektionssystem,
- anderen ähnlichen Systemen.

11. Das Gerät gemäß einem oder mehreren der vorangehenden Ansprüche, **dadurch gekennzeichnet, dass** die Mittel (42) zum Erzeugen akustischer Rückmeldungen mindestens aus einem der folgenden Elemente bestehen:
- mindestens einem Lautsprecher,
- mindestens einem einkanaligen System,
- Mono- oder Stereo-Kopfhörern,
- einem Positionsaudiosystem.

12. Das Gerät gemäß einem oder mehreren der vorangehenden Ansprüche, **dadurch gekennzeichnet, dass** die Mittel (43) zum Erzeugen der taktilen Rückmeldung mindestens aus einem der folgenden Elemente bestehen:
- Mitteln vibrierender Art,
- Mitteln zur Kraft- oder Druckrückmeldung,
- einer Reihe, einem Netz oder einer Anordnung von Elektrostimulatoren zur Anwendung auf besonders empfindliche Teile des Körpers.

13. Das Gerät gemäß Anspruch 12, **dadurch gekennzeichnet, dass** die Mittel vibrierender Art Vibrationsquellen sind bedingt durch pneumatische oder hydraulische Druckwellen oder exzentrische Elemente, die von Elektromotoren angetrieben werden.

14. Das Gerät gemäß einem oder mehreren der vorangehenden Ansprüche, **dadurch gekennzeichnet, dass** die Mittel (24) zum Verarbeiten, Speichern und Zusammenfassen eine Zentraleinheit (24) umfassen, die direkt oder ferngesteuert werden kann.

15. Das Gerät gemäß dem vorangehenden Anspruch, **dadurch gekennzeichnet, dass** die Fernsteuerung mittels eines Datenkommunikationsnetzes, das auch drahtlos sein kann, erfolgt.

16. Das Gerät gemäß einem oder mehreren der vorangehenden Ansprüche, **dadurch gekennzeichnet, dass** es mit Mitteln zum Verbinden mit einem oder mehreren tragbaren Speichern (48), die dem Benutzer zur individualisierten Benutzung des Geräts zur Verfügung stehen, ausgestattet ist.

## Revendications

1. Appareil pour l'entraînement moteur et l'exercice du corps humain, comportant :
- des moyens (11, 12, 14, 16, 17) pour acquérir des données biophysiologiques et des paramètres de mouvements associés à une action humaine du type qui peut être commandé de manière volontaire par l'individu qui l'exécute,
- des moyens (24) pour traiter, mémoriser et récapituler les données et les paramètres acquis,
- et des moyens (41, 42, 43) pour renvoyer au moins deux retours d'informations relatives aux données et aux paramètres acquis, ou acquis et traités, appropriés pour faciliter la correction de l'action humaine volontaire étant exécutée, lesdits moyens de retour d'informations (41,42, 43) étant conçus pour utiliser au moins un canal sensoriel,
lesdits moyens d'acquisition (11, 12, 14, 16, 17) comportent au moins un dispositif (11) pour acquérir des paramètres associés à la position et au mouvement de l'utilisateur ou d'une partie du corps de l'utilisateur,
lesdits moyens (24) pour traiter, mémoriser et récapituler les données et les paramètres acquis comportent au moins :
- un ou plusieurs systèmes pour fournir des environnements virtuels,
- un ou plusieurs systèmes pour une interaction virtuelle avec des formes ou des objets réels ou des formes virtuelles qui sont fixes ou mobiles à l'intérieur d'environnements virtuels,
lesdits moyens pour renvoyer au moins deux retours comportent au moins
- des moyens (41) pour générer des retours visuels,
- des moyens (42) pour générer des retours acoustiques,
lesdits moyens (41) pour générer des retours visuels comportent :
- un système pour afficher des formes multidimensionnelles, incluant des formes anthropomorphiques, lesquelles sont adaptées pour représenter ledit utilisateur, et/ou tout autre individu automatisé ou virtuel interagissant, et/ou tout autre individu réel interagissant qui est l'utilisateur d'un système équivalent par l'intermédiaire d'un autre appareil,
- un système pour le rendu tridimensionnel en temps réel d'objets, de manière facultative ayant également des propriétés cinématiques.

2. Appareil selon la revendication 1, **caractérisé en ce que** lesdits moyens d'acquisition sont constitués d'au moins un dispositif de manière sélective parmi :
- un dispositif (12) pour acquérir des données électromyographiques,
- un dispositif (14) pour acquérir des données électroencéphalographiques,
- un dispositif (16) pour acquérir des images,
- un dispositif (17) pour acquérir des données associées à une activité cardiaque et à la pression des vaisseaux sanguins.

3. Appareil selon l'une ou plusieurs des revendications précédentes, **caractérisé en ce que** ledit dispositif (11) pour acquérir des paramètres associés à la position et au mouvement d'au moins une partie du corps de l'utilisateur comporte de manière sélective :
un ou plusieurs capteurs de mouvement et de position électromagnétiques, ou à un ou plusieurs capteurs de mouvement et de position à ultrasons, ou un dispositif stéréophotogrammétrique ayant des marqueurs optiques (22) et une analyse d'images, ou un ou plusieurs systèmes du type accélérométrique, un ou plusieurs capteurs piézo-électriques ou à résistance variable

4. Appareil selon l'une des revendications précédentes, **caractérisé en ce que** ledit dispositif (11) pour acquérir des paramètres relatifs à la position et au mouvement d'au moins une partie du corps de l'utilisateur est associé à des moyens pour étudier l'équilibre du corps.

5. Appareil selon la revendication 4, **caractérisé en ce que** lesdits moyens pour étudier l'équilibre du corps sont constitués par une plate-forme dynamométrique (25), par des plates-formes stabilométriques, des plates-formes de force et analogue.

6. Appareil selon l'une ou plusieurs des revendications précédentes, **caractérisé en ce que** lesdits moyens (11) pour acquérir des paramètres de mouvement et de position sont associés à des machines de gymnastique.

7. Appareil selon l'une ou plusieurs des revendications précédentes, **caractérisé en ce que** lesdits moyens (11) pour acquérir des paramètres de mouvement et de position sont associés à des machines à commande numérique pour l'assistance au mouvement et la réadaptation.

8. Appareil selon l'une ou plusieurs des revendications précédentes, **caractérisé en ce que** lesdits moyens (24) pour traiter, mémoriser et récapituler les données et les paramètres acquis comportent au moins un système de manière sélective parmi :
- un ou plusieurs systèmes (27) pour traiter les signaux, les données et les paramètres,
- un ou plusieurs systèmes (28) pour préparer des algorithmes analytiques pour les données acquises,
- un ou plusieurs systèmes (31) pour mémoriser des signaux, des données acquises, des données et des paramètres traités,
- un ou plusieurs systèmes de transmission de données (32),
- un ou plusieurs systèmes (33, 36, 36a, 37, 37a, 38, 38a, 39, 39a, 40, 40a) pour gérer la sortie de données,
- un ou plusieurs systèmes d'analyse de signaux (34),
- un ou plusieurs systèmes d'analyse de données (35),
- un ou plusieurs systèmes d'interface utilisateur,
- un ou plusieurs systèmes pour virtualiser des objets et des éléments en général qui n'existent pas.

9. Appareil selon l'une ou plusieurs des revendications précédentes, **caractérisé en ce que** lesdits moyens (41, 42, 43) pour renvoyer au moins deux retours comportent :
- des moyens (43) pour générer un retour tactile.

10. Appareil selon l'une ou plusieurs des revendications précédentes, **caractérisé en ce que** lesdits moyens (41) pour générer des retours visuels sont constitués par au moins l'un parmi :
- un écran d'affichage bidimensionnel ou tridimensionnel,
- des lunettes stéréoscopiques,
- un système de projection d'images bidimensionnelles ou tridimensionnelles,
- d'autres systèmes similaires.

11. Appareil selon l'une ou plusieurs des revendications précédentes, **caractérisé en ce que** lesdits moyens (42) pour générer des retours acoustiques sont constitués par au moins l'un parmi :
- au moins un haut-parleur,
- au moins un système monaural,
- des écouteurs mono ou stéréo,
- un système audio positionnel.

12. Appareil selon l'une ou plusieurs des revendications précédentes, **caractérisé en ce que** lesdits moyens (43) pour générer un retour tactile sont constitués par au moins l'un parmi :
- des moyens du type vibratoire,
- des moyens pour un retour en force ou en pression,
- une série, un réseau ou une rangée de stimulateurs électriques appliqués à des parties particulièrement sensibles du corps.

13. Appareil selon la revendication 12, **caractérisé en ce que** lesdits moyens du type vibratoire sont des sources de vibrations dues à des ondes à pression pneumatique ou hydraulique ou des éléments excentriques actionnés par des moteurs électriques.

14. Appareil selon l'une ou plusieurs des revendications précédentes, **caractérisé en ce que** lesdits moyens (24) pour traiter, mémoriser et récapituler comportent une unité centrale de traitement (24) laquelle peut être commandée directement ou par l'intermédiaire d'une liaison à distance.

15. Appareil selon la revendication précédente, **caractérisé en ce que** ladite commande à distance s'effectue par l'intermédiaire d'un réseau de communication de données qui peut également être sans fil.

16. Appareil selon l'une ou plusieurs des revendications précédentes, **caractérisé en ce que** il est muni de moyens pour s'interfacer avec une ou plusieurs mémoires portables (48) lesquelles sont mises à disposition de l'utilisateur en vue d'une utilisation personnalisée de l'appareil.
